(19) Europäisches Patentamt
European Patent Office
Office européen des brevets

(11) Veröffentlichungsnummer: **0 010 588**
**B1**

(12) # EUROPÄISCHE PATENTSCHRIFT

(45) Veröffentlichungstag der Patentschrift: **13.03.85**

(21) Anmeldenummer: **79103164.4**

(22) Anmeldetag: **27.08.79**

(51) Int. Cl.⁴: **A 01 N 25/32**, C 07 C153/05, C 07 C154/00, C 07 C155/02, C 07 C161/00

(54) **Schwefelhaltige Oxim-Verbindungen und ihre Verwendung zum Schutz von Kulturpflanzen.**

(30) Priorität: **28.08.78 CH 9081/78**

(43) Veröffentlichungstag der Anmeldung: **14.05.80 Patentblatt 80/10**

(45) Bekanntmachung des Hinweises auf die Patenterteilung: **13.03.85 Patentblatt 85/11**

(84) Benannte Vertragsstaaten: **AT BE CH DE FR GB IT NL**

(56) Entgegenhaltungen:
**DE-A- 2 119 700**
**DE-B- 1 141 487**
**DE-B- 1 567 075**
**FR-A- 2 322 861**

(73) Patentinhaber: **CIBA-GEIGY AG**
**Postfach**
**CH-4002 Basel (CH)**

(72) Erfinder: **Sturm, Elmar, Dr.**
**Ziegelbündtenweg 20 B**
**CH-4147 Aesch (CH)**
Erfinder: **Schempp, Heinrich, Dr.**
**Im Baumgarten 5**
**CH-4144 Arlesheim (CH)**
Erfinder: **Martin, Henry, Dr.**
**Jupiterstrasse 17**
**CH-4123 Allschwil (CH)**

(74) Vertreter: **Zumstein, Fritz sen., Dr. et al**
**Bräuhausstrasse 4**
**D-8000 München 2 (DE)**

**EP 0 010 588 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft schwefelhaltige Oxim-Verbindungen, welche der Formel I entsprechen, sowie Mittel, welche solche Oxim-Verbindungen als Wirkstoffe enthalten, ferner die Anwendung solcher Oxim-Verbindungen zum Schutz von Kulturpflanzen vor der phytotoxischen Wirkung von aggressiven Herbiziden, und Vermehrungsgut von Kulturpflanzen, welches mit solchen Oxim-Verbindungen behandelt worden ist.

Die für diesen Zweck einsetzbaren Wirkstoffe entsprechen der folgenden Allgemeinformel I

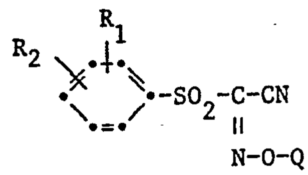

(I)

worin $R_1$ und $R_2$ unabhängig voneinander Wasserstoff, Halogen oder $C_1$-$C_4$-Alkyl bedeuten und Q die Acetamidgruppe, eine $C_1$-$C_4$-Alkoxycarbonylgruppe, eine $C_2$-$C_4$-Alkenylgruppe, eine N-($C_1$-$C_4$-Alkyl)-Carbamoylgruppe, eine Benzoylgruppe, die gegebenenfalls ein- bis dreifach durch gleiche oder verschiedene Substituenten aus der Gruppe Halogen, $C_1$-$C_4$-Alkyl und $C_1$-$C_4$-Alkoxy substituiert ist, oder eine 2-Furoylgruppe darstellt.

In der Formel I ist unter Halogen Fluor, Chlor, Brom oder Jod zu verstehen.

Der Ausdruck $C_1$-$C_4$-Alkyl allein oder als Teil eines Substituenten steht für Methyl, Aethyl, n-Propyl, Isopropyl, n-Butyl, Isobutyl, sec. Butyl oder tert. Butyl.

Die neuen Verbindungen der Formel I stellen Oximäther, Oximester, Oximcarbamate und Oximcarbonate dar.

Schwefelhaltige Oxim-Verbindungen mit fungizider Wirkung sind bereits beschrieben worden. In der DE-OS 2 119 700 werden 2-Imino-2-sulfonyl-acetamid-Derivate mit fungizider Wirkung offenbart, und in der DE-AS 1 141 487 sind fungizide Mittel beschrieben, welche als Aktivsubstanzen α-(Phenylsulfonyl)-α-(oxyimino)-acetonitril-Derivate enthalten, in denen die Oxyiminogruppe durch ein Natriumkation oder durch eine Methyl- oder Isopropylgruppe substituiert ist.

Die Oxime der Formel I eignen sich hervorragend, Kulturpflanzen wie z. B. Kulturhirse, Reis, Mais, Getreidearten (Weizen, Roggen, Gerste, Hafer), Baumwolle, Zuckerrüben, Zuckerrohr und Soja vor dem Angriff von pflanzenagressiven Herbiziden verschiedenster Stoffklassen, wie z. B. Triazinen, Phenylharnstoffderivaten, Carbamaten, Thiolcarbamaten, Halogenacetaniliden, Halogenphenoxyessigsäureestern, subst. Phenoxyphenoxyessigsäureestern und -propionsäureester, subst. Pyridinoxyphenoxy-essigsäureestern und -propionsäureestern sowie Benzoesäurederivaten zu schützen, sofern diese nicht oder nicht genügend selektiv wirken, also neben den zu bekämpfenden Unkräutern auch die Kulturpflanzen schädigen. Die Erfindung betrifft sowohl Mittel, welche diese Oximäther der Formel I zusammen mit Herbiziden enthalten, als auch Mittel, welche diese Oximäther der Formel I als einzige Aktivkomponente enthalten. Pflanzenschutzmittel, die ein Gegenmittel der Formel I (auch Antidote oder Safener genannt) enthalten, können ohne gleichzeitige Anwesenheit des abzuschwächenden Herbizids hergestellt, in den Handel gebracht oder verwendet werden. Eine wichtige Anwendungsmöglichkeit ist die Saatbeizung, die zu einem von der Anwendung des Herbizids ganz unabhängigen Zeitpunkt vorgenommen wird. Ein anderes Einsatzgebiet ist die Behandlung eines Bodens, in dem noch Reste eines Herbizids der letzten Anbausaison vorhanden sind, das die vorgesehene neuen Pflanzenkultur schädigen könnte.

Die Antidote-Eigenschaft ist eine von der Kulturpflanze und von dem in der Wirkung selektiv abzuschwächenden Herbizid unabhängige Stoffeigenschaft, die einem Präparat der Formel I inhärent ist, jedoch erst beim Zusammenwirken der drei Komponenten Antidote-Herbizid Pflanze sichtbar zu Tage tritt. Aehnlich einer pestizid wirkenden Chemikalie, deren Pestizid-Wirkung erst bei Anwesenheit eines Schädlings sichtbar wird, erfordert auch der Nachweis der Safener-Wirkung die Anwesenheit der an der Wirkung beteiligten anderen beiden Komponenten, nämlich des Herbizids und der Kulturpflanze. Dies unterscheidet ein formuliertes Safener-Mittel grundsätzlich von einem synergistisch wirkenden Zwei- oder Dreikomponentengemisch, bei dem gleichzeitig alle Wirkstoffkomponenten vorliegen und sämtlich in die gleiche Wirkungsrichtung zielen.

Als Gegenmittel oder Antidote sind schon verschiedene Stoffe vorgeschlagen worden, welche befähigt sind, die schädigende Wirkung eines Herbizids auf die Kulturpflanze spezifisch zu antagonisieren, d. h. die Kulturpflanze zu schützen, ohne dabei die Herbizidwirkung auf die zu bekämpfenden Unkräuter merklich zu beeinflussen. Dabei kann ein solches Gegenmittel je nach seinen Eigenschaften zur Vorbehandlung des Saatgutes der Kulturpflanze (Beizung des Samens oder der Stecklinge) oder vor der Saat in die Saatfurchen oder als Tankmischung für sich allein oder zusammen mit dem Herbizid vor oder nach dem Auflaufen der Pflanzen verwendet werden.

So schreibt die GB-PS 1'277'557 die Behandlung von Samen bzw. Sprösslingen von Weizen und Sorghum mit gewissen Oxamsäureestern und Amiden vor dem Angriff durch N-Methoxymethyl-2',6'-diäthyl-chloracetanilid (Alachlor). Andere Literaturstellen (DE-OS 1'952 910, DE-OS 2'245'471, FR-PS 2'021'611) schlagen Gegenmittel zur Behandlung von Getreide, Mais- und Reis-Samen zum Schutz

gegen den Angriff herbizider Thiocarbamate vor. In der DE-OS 1'567'075 und der US-PS 3'131'509 werden Hydroxy-amino-acetanilide und Hydantoine für den Schutz von Getreidesamen gegenüber Carbamaten wie z. B. IPC, CIPC vorgeschlagen. In der weiteren Entwicklung haben sich alle diese Präparate jedoch als ungenügend erwiesen.

Ueberraschenderweise besitzen Oxime der Formel I die Eigenschaft, Kulturpflanzen vor dem Angriff pflanzenaggressiver Herbizide der verschiedensten Stoffklassen zu schützen, darunter 1,3,5-Triazinen, 1,2,4-Triazinonen, Phenylharnstoffderivaten, Carbamaten, Thiolcarbamaten, Phenoxyessigsäureestern, Phenoxypropionsäureestern, Halogenacetaniliden, Halogenphenoxyessigsäureestern, subst. Phenoxyphenoxyessigsäureestern und -propionsäureestern, subst. Pyridinoxyphenoxy-essigsäureestern und -propionsäureestern und Benzoesäurederivaten, sofern diese nicht oder ungenügend kulturentolerant sind.

Ein solches Gegenmittel oder Antidote der Formel I kann je nach Anwendungszweck zur Vorbehandlung des Saatgutes der Kulturpflanze (Beizung des Samens oder der Stecklinge) eingesetzt oder vor oder nach der Saat in den Erdboden gegeben werden oder aber für sich allein oder zusammen mit dem Herbizid vor oder nach dem Auflaufen der Pflanzen appliziert werden. Die Behandlung der Pflanze oder des Saatguts mit dem Antidote kann daher grundsätzlich unabhängig vom Zeitpunkt der Applikation des phytotoxischen Herbizids erfolgen. Sie kann jedoch auch gleichzeitig durchgeführt werden (Tankmischung). Vorauflauf-Behandlung schliesst sowohl die Behandlung der Anbaufläche vor der Aussaat (ppi = « pre plant incorporation ») als auch die Behandlung der angesäten, aber noch nicht bewachsenen Anbauflächen ein.

Die Aufwandmengen des Antidotes im Verhältnis zum Herbizid richten sich weitgehend nach der Anwendungsart. Sofern eine Feldbehandlung vorgenommen wird, verhalten sich die Mengen von Antidote der Formel I zu phytotoxischem Herbizid wie 1 : 100 bis 1 : 5, bevorzugt 1 : 20 bis 1 : 1. Bei Samenbeizung und ähnlichen gezielten Schutzmassnahmen werden jedoch weit geringere Mengen Antidote im Vergleich mit den z. B. später pro Hektar Anbaufläche verwendeten Herbizidmengen benötigt (z. B. ca. 1 : 3 000 bis 1 : 1 000). In der Regel stehen protektive Massnahmen wie Samenbeizung mit einem Antidote der Formel I und mögliche spätere Feldbehandlung mit Herbiziden nur in losem Zusammenhang. Vorbehandeltes Saat- und Pflanzengut kann später in Landwirtschaft, Gartenbau und Forstwirtschaft mit unterschiedlichen Herbiziden in Berührung kommen.

Kulturpflanzen seien im Rahmen vorliegender Erfindung alle Pflanzen, die in irgendeiner Form Ertragsstoffe produzieren (z. B. Samen, Wurzeln, Stengel, Knollen, Blätter, Blüten, Inhaltsstoffe wie Oele, Zucker, Stärke und Eiweiss) und zu diesem Zweck angebaut und gehegt werden. Zu diesen Pflanzen gehören beispielsweise sämtliche Getreidearten, Mais, Reis, Edelhirse, Soja, Bohnen, Erbsen, Kartoffeln, Gemüse, Baumwolle, Zuckerrüben, Zuckerrohr, Erdnüsse, Tabak, Hopfen, dann jedoch auch Zierpflanzen, Obstbäume sowie Bananen-, Kakao- und Naturkautschuk-Gewächse. Diese Aufzählung stellt keine Limitierung dar. Grundsätzlich lässt sich ein Antidote überall dort einsetzen, wo eine Kulturpflanze vor der Phytotoxizität eines Herbizids geschützt werden soll.

Die Erfindung bezieht sich auch auf ein Verfahren zum Schutz von Kulturpflanzen vor aggressiven (phytotoxischen) Herbiziden, indem ein als Antidote wirkendes Oximderivat der Formel I wahlweise vor oder nach Applikation des Herbizids oder auch gleichzeitig mit dem Herbizid angewendet wird.

Die Erfindung bezieht sich ferner auf das Vermehrungsgut solcher Kulturpflanzen, das protektiv mit einem Oximderivat der Formel I behandelt wurde. Unter dem Begriff « Vermehrungsgut » sind alle generativen Pflanzenteile zu verstehen, die zur Vermehrung der Kulturpflanze eingesetzt werden können. Dazu zählen Samenkörner (Saatgut im engeren Sinne), Wurzeln, Früchte, Knollen, Rhizome, Stengelteile, Zweige (Stecklinge) und andere Pflanzenteile. Es zählen aber auch angekeimte Pflanzen und Jungpflanzen dazu, die nach der Ankeimung oder dem Auflaufen weiterverpflanzt werden sollen. Solche Jungpflanzen lassen sich durch eine vollständige oder partielle Tauchbehandlung vor dem Weiterverpflanzen gezielt schützen.

Verbindungen der Formel I lassen sich nach mehreren Verfahren aus den freien Oximen herstellen, die nachstehend schematisch wiedergegeben sind :

Die Herstellung der Verbindungen der Formel I erfolgt nach an sich bekannten Verfahren ; siehe z. B. J. f. prakt. Chemie *66* S. 353 ; Liebigs Ann. *250*, 165.

Durch Kondensation von substituierten Oximino-Verbindungen bzw. ihrer Salze, besonders ihrer Alkalisalze oder Ammoniumsalze mit reaktionsfähigen Partnern entstehen die Verbindungen der Formel I (Organic Reaktions 1953, Band *7*, Seite 343 und 373).

Je nach der Bedeutung des Substituenten Q in der Formel I eutstehen Oximäther, Oximester, Oximcarbamate oder Oximcarbonate.

Die Kondensation der substituierten Oximino-Verbindungen mit reaktionsfähigen Alkyl- oder Alkenyl-Derivaten erfolgt bei der Verätherung zweckmässig in Form ihrer Salze, besonders ihrer Alkalimetallsalze oder Ammoniumsalze wie im folgenden ausgewählten Beispiel dargestellt wird :

a) 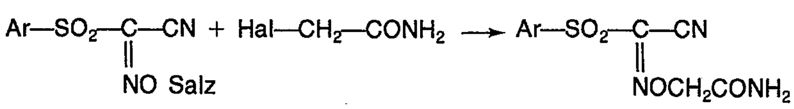

Oximäther der Formel I

(Oxim-alkancarbonsäureamide der Formel I)

Die Bildung der wie in den Ansprüchen angegeben gegebenenfalls substituierten Oximester der Formel I erfolgt zweckmässig ebenfalls mit den Oximsalzen. Es können jedoch auch die freien Oxime in Gegenwart säurebindender Mittel mit geeigneten Acylierungsmitteln verestert werden, wie das folgende Schema zeigt.

b) 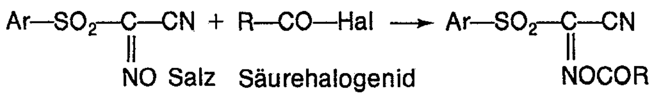


Acyl-oxim der Formel I (R = gegebenenfalls substituiertes Phenyl ; 2-Furyl)

Zur Herstellung dieser Ester können mit Vorteil die reaktionsfähigen Carbonsäurehalogenide wie auch die Carbonsäureanhydride verwendet werden.

Die Herstellung der Oxim-carbamate der Formel I erfolgt zweckmässig durch Umsetzen der freien Oxime mit Isocyanaten oder der Oximsalze mit Halogenkohlensäure-mono-$C_1$-$C_4$-Alkyl-substituierten Amiden, wie an den untenstehend ausgewählten Beispielen dargestellt werden soll :

c) 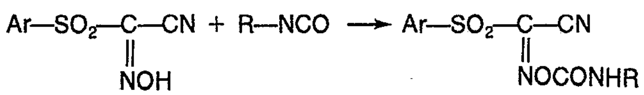

monosubstituiertes Oximcarbamat der Formel I (R = $C_1$-$C_4$-Alkyl)

Zur Herstellung der Carbamate eignen sich die Carbaminsäurehalogenide, ganz besonders aber die Isocyanate. Als Isocyanate seien z. B. genannt :

Methylisocyanat
Aethylisocyanat
iso-Propylisocyanat
t-Butylisocyanat
n-Propylisocyanat
sec. Butylisocyanat
iso-Butylisocyanat

Ein geeignetes Carbamoylhalogenid ist beispielsweise das Methylcarbamoylchlorid.

Zur Herstellung von Oximcarbonaten der Formel I werden Oximsalze mit Halogenkohlensäureestern umgesetzt. Man kann ebenfalls die freien Oxime in Gegenwart säurebindender Mittel mit Halogenkohlensäureestern umsetzen, (siehe das folgende Reaktionsschema, welches diese Umsetzung erläutern soll) :

d) 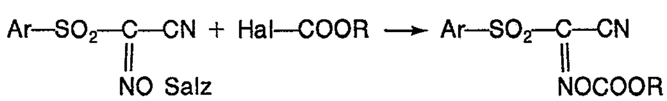


Oximcarbonat der Formel I (R = $C_1$-$C_4$-Alkyl)

Weitere allgemeine Reaktionsmöglichkeiten welche zu Verbindungen der Formel I führen, sind zum Beispiel die Umsetzung von Oximkohlensäurehalogeniden mit $C_1$-$C_4$-Alkyl-Aminen oder, $C_1$-$C_4$-Alkoholen, wobei entweder Oximcarbamate oder Oximcarbonate gebildet werden. Auch solche Reaktionen sollen durch die folgenden Beispiele formelmässig veranschaulicht werden.

e) 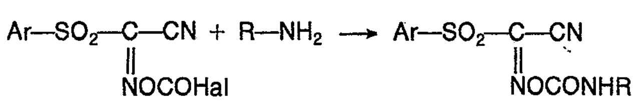

Oximcarbamat der Formel I (R =$C_1$-$C_4$-Alkyl)

f) 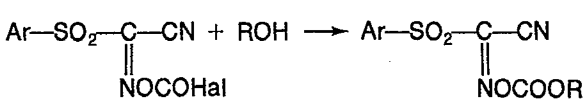

Oximcarbonat der Formel I (R = $C_1$-$C_4$-Alkyl)

Die zur Herstellung der Verbindungen der Formel I notwendigen Ausgangsmaterialien, nämlich die Oxime bzw. deren Salze sind bekannt. (Arch. Pharm. *246* 631) Durch Umsetzung der Arylsulfonacetonitrile wie deren Kernsubstitutionsprodukte mit Alkylnitrilen Na-äthylat entstehen die unsubstituierten bzw. kernsubstituierten Oxime bzw. deren Natriumsalze.

Unter den bevorzugten Oximnitrilen befinden sich die folgenden leicht zugänglichen Ausgangsprodukte :

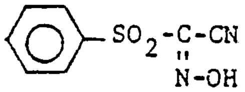

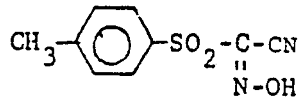

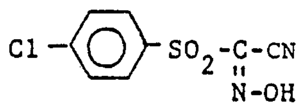

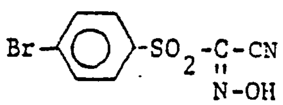

Als Lösungsmittel zur Gewinnung der Verbindungen der Formel I eignen sich prinzipiell alle Vertreter, die sich unter den Bedingungen der Reaktion indifferent erhalten. Beispielsweise Kohlenwasserstoffe, vor allem aber polare Lösungsmittel wie Acetonitril, Dioxan, Cellosolve, DMF, aber auch Ketone wie Methyläthylketon usw.

Die Temperaturen liegen im Bereich von – 20° bis ca. 150°, bevorzugt zwischen 20° und 60°.

Als halogenwasserstoffabspaltende Mittel können Basen wie tert. Amine (Triäthylamin, Triäthylendiamin, N-Methylpiperidin, N-Methylmorpholin, Dimethylanilin) eingesetzt werden.

In einigen Fällen genügt eine Suspendierung von wasserfreier $Na_2CO_3$ oder wasserfreier $K_2CO_3$ in wasserfreiem Reaktionsmedium.

Oxime liegen in zwei stereoisomeren Formen, der syn. und anti-Form, vor. Alle genannten Endprodukte entsprechen der Formel I und können in beiden Formen rein oder als Gemische vorliegen. Im Rahmen der vorliegenden Beschreibung sind demgemäss beide stereoisomere Formen für sich und als Gemische in beliebigem gegenseitigen Mischungsverhältnis zu verstehen.

Eine der bevorzugten Untergruppen von Antidoten oder Safenern sind Verbindungen der Formel

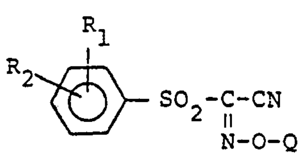

worin $R_1$ Wasserstoff, Chlor, Methyl oder Aethyl und $R_2$ Wasserstoff bedeuten und Q —$CH_2CONH_2$, —$COOCH_3$, eine $C_2$-$C_4$-Alkenylgruppe, eine N-Methylcarbamoylgruppe, eine Benzoylgruppe, die durch zwei Chloratome und eine Methoxygruppe substituiert ist, oder eine 2-Furoylgruppe darstellt.

Als wichtige Einzelverbindungen im Rahmen der vorgenannten Stoffgruppe von Antidoten sind die weiter unten in der Tabelle genannten Verbindungen Nr. 3, 4, 5, 8, 11, 13 und 14.

Die nachfolgenden Beispiele erläutern die Herstellung der Verbindungen der Formel I. Anschliessend befindet sich eine Tabelle von in analoger Weise zu diesen Beispielen hergestellten Verbindungen. Die Temperaturen sind in Celsiusgraden gegeben, Prozentangaben beziehen sich auf das Gewicht.

Beispiel 1

1/10 Mol 4-Bromphenylsulfonylglyoxylonitriloxim-natrium wird fein gemahlen und in wenig trockenem Aether suspendiert und mit der berechneten Menge frisch destilliertem Benzoylchlorid versetzt und

längere Zeit auf dem Wasserbad erhitzt. Die ausgeschiedene Masse wird aus Chloroform umkristallisiert, wobei man das bei 170-172° schmelzende Produkt der Formel

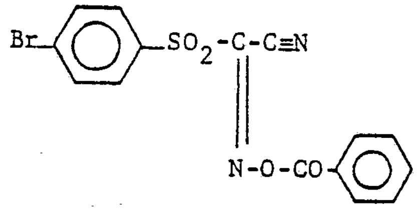

erhält.
(Verbindung No. 1)

Beispiel 2

10 g 4-Methylphenylsulfonyl-glyoxylonitriloxim-natrium wird fein gemahlen und in 50 ml Acetonitril suspendiert. Nach Zugabe von 24,2 g Allylbromid wird 2 Stunden auf 50-60° Badtemperatur gehalten. Danach wird eingedampft und der halbfeste Rückstand mit Wasser verrührt, wobei das Produkt als beige gefärbtes Kristallisat ausfällt. Ausbeute 9,7 g (91,5 % Ausbeute) Smp. 34-36°. Das Produkt entspricht der Formel

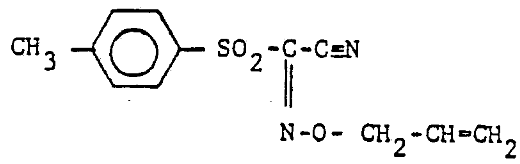

(Verbindung No. 2) und enthält 11,6 % Schwefel, ber : 12, 13 %).

Beispiel 3

0,04 Mol Na-Salz des 4-Methylphenylsulfonylglyoxylonitril-oxim werden fein gemahlen und in 50 ml Acetonitril suspendiert. Dazu werden 0,04 Mol Jodacetamid zugegeben und 2 Stunden auf 50-60° gehalten und dann bei dieser Temperatur eingedampft. Der Rückstand wurde mit Methylenchlorid versetzt, dann filtriert mit Tiorkohle behandelt und eingedampft. Smp. 115-122°. Aus Methanol/$H_2O$ umkristallisert. Smp. 130-132° und entspricht der Formel

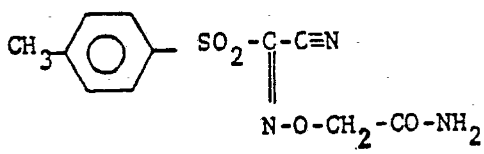

(Verbindung No. 11)

Beispiel 4

10 g Phenylsulfonylglyoxylonitril-oxid (0,05 Mol) werden in 100 cc Acetonitril gelöst, mit einer Spatelspitze DABCO versetzt und 10,8 g Methylisocyanat (0,2 Mol) zugegeben. Es wird eine leichte Temperaturzunahme von 23° auf 27° beobachtet. Es wurde 5 Stunden ein 50-60° gehalten, worauf eine feine Suspension entstand. Das Reaktionsgemisch wurde in ca. 500 ml Eiswasser gerührt und das fein kristallin ausgefallene Produkt abgenutscht und mit Wasser gewaschen. Es schmilzt bei 120-123° und entspricht durch Verfahren und Analyse der Formel

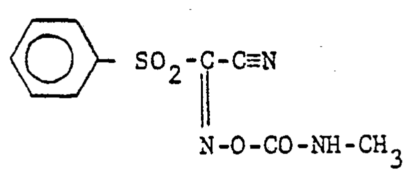

(Verbindung No 3) mit folgender Analyse :

Berechnet C 44,94 % gefunden C 45,4 %
Berechnet H 3,39 % gefunden H 3,6 %
Berechnet N 15,72 % gefunden N 15,7 %
Berechnet S 12,00 % gefunden S 11,9 %

In analoger Weise zu diesen Beispielen wurden die in der folgenden Tabelle aufgeführten Verbindungen erhalten.

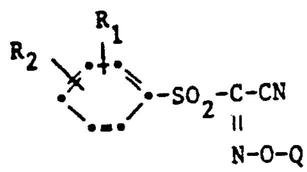

,

| Verb. Nr. | Ar | Q | physikalische Konstante |
|---|---|---|---|
| 1 | 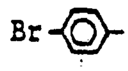 | 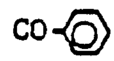 | Smp. 170-172° |
| 2 | 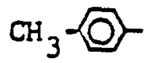 | $CH_2$-CH=$CH_2$ | Smp. 34-36° |
| 3 | 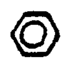 | CO-NH$CH_3$ | Smp. 120-123° |
| 4 | 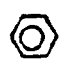 | 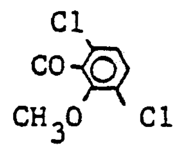 | Smp. 109-111° |
| 5 | 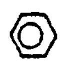 | $CH_2CONH_2$ | Smp. 129-130° |
| 6 | 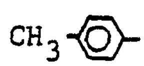 | $CONHCH_3$ | Smp. 100-104° |
| 7 | 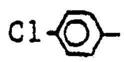 | $CONHCH_3$ | Smp. 125-128° |
| 8 | 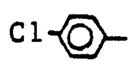 | $COOCH_3$ | Smp. 114-116° |
| 9 | 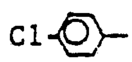 | 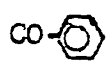 | |
| 10 | 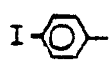 | 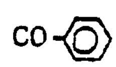 | |
| 11 | 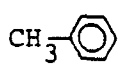 | $CH_2CONH_2$ | Smp. 130-132 |
| 12 | 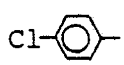 | $CH_2CONH_2$ | Smp. 148° (Z.) |
| 13 | 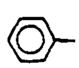 | $CH_2CH=CH-CH_3$ | Oel |
| 14 | 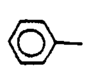 | 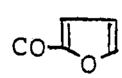 | Smp. 150-2° |
| 15 | 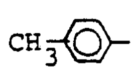 | $-CH_2CH=CH-CH_3$ | Oel |

Die für Kulturpflanzen gegen starke Herbizide antagonistische Wirkung wurde durch folgende Versuche nachgeprüft:

Vorauflauf-Antidote-Test (Grundtest)

Allgemeine Methodik:

Kleine Blumentöpfe (oberer Ø 6 cm) werden mit Gartenerde gefüllt, in die die Pflanzenkultur eingesät, bedeckt und leicht festgedrückt wird. Dann wird die als Antidote zu prüfende Substanz als (aus einem Spritzpulver erhaltene) verdünnte Lösung in einer Menge aufgesprüht, die 4 kg AS/ha entspricht. Unmittelbar danach wird in entsprechender Weise das Herbizid aufgesprüht. Nach 18 Tagen Stehen bei ca. 20-23 °C und 60-70 % relativer Luftfeuchtigkeit wird nach einer linearen Skala von 1 bis 9 ausgewertet, wobei 1 totale Pflanzenschädigung und 9 unbeeinträchtigter Gesundheitszustand bedeuten. Als Kontrolle dienen Pflanzen ohne Antidote-Schutz.

Es wurden verwendet:

1) 1,5 kg AS/ha α-[4-(p-Trifluormethylphenoxy)-phenoxy]-propionsäure-n-butoxyäthylester in Mais der Sorte « Orla 264 ».

2) 1,5 kg AS/ha Metolachlor = N-(1-Methyl-2-methoxyäthyl)-N-chloracetyl-2-äthyl-6-methylanilin in Sorghum-Hirse der Sorte « Funk G-522 ».

3) 2,0 kg AS/ha Prometryn = 2,4-bis(Isopropylamino)-6-methylthio-s-triazin in Soja.

4) 2,0 kg AS/ha 4-Aethylamino-6-tert.butylamino-2-chlor-s-triazin in Weizen der Sorte « Farnese ».

5) 4,0 kg AS/ha Prometryn = 2,4-bis(Isopropylamino)-6-methylthio-s-triazin in Sorghum-Hirse der Sorte « Funk G-522 ».

6) 2,0 kg AS/ha α-[4-(p-Trifluormethylphenoxy)-phenoxy]-propionsäure-n-butoxyäthylester in Gerste der Sorte « Mazurka ».

Mit den Verbindungen der Formel I erhält man folgendes Resultat:

| Testvariante | Verb. Nr. | Note des Herbizideinflusses (ohne/mit Antidote) | |
|---|---|---|---|
| 5 | 8 | 6 | 8 |
| 5 | 11 | 6 | 8 |
| 3 | 13 | 3 | 5 |

Vorauflauf-Antidote Test in Nährlösung (Reis)

Es wird eine Hewitt-Nährlösung hergestellt, die zusätzlich 10 ppm des zu prüfenden Antidotes enthält.

Reissamen der Sorte « IR-8 » wird in inertes Füllmaterial (gekörntes Zonolith) gesät, das sich in einem an der Unterseite durchlöcherten Plastik-Blumentopf (oberer Ø 6 cm) befindet. Dieser wird in einen zweiten durchsichtigen Plastik-Blumentopf (oberer Ø 7 cm) gestellt, in dem sich ca. 50 ml der vorbereiteten Nährlösung befinden, die nunmehr kapillar im Füllmaterial des kleineren Topfes aufsteigt und Samen und keimende Pflanze benetzt. Täglich wird der Flüssigkeitsverlust mit reiner Hewit-Nährlösung auf 50 ml ergänzt. Nach 15 Tagen werden die Reispflanzen im 2- bis 21/2-Blattstadium in rechteckige Plastiktöpfe (8 × 8 cm, 10 cm Höhe) verpflanzt, die mit 500 ml sumpfignasser Erde gefüllt sind. Am nächsten Tag wird der Wasserstand darin auf 1-2 cm über Boden-Niveau erhöht. 4 Tage nach Verpflanzung gibt man das Herbizid 2-Aethylamino-4-(1,2-dimethyl-n-propylamino)-6-methylthio-s-triazin in Granulatform und in einer Menge zu, die umgerechnet 0,75 kg AS/ha beträgt. 3 Wochen nach Herbizidzugabe wird nach einer linearen Skala von 1 bis 9 ausgewertet, wobei 1 totale Pflanzenschädigung und 9 unbeeinträchtigter Gesundheitszustand bedeuten. Die parallel angewandte Kontroll-Lösung enthält keinen Antidote-Zusatz.

Die Verbindung Nr. 2 bewirkt die folgende deutliche Abschwächung der phytotoxischen Wirkungen des Triazin-Herbizides.

| Verb. Nr. | Note des Herbizideinflusses (ohne/mit Antidote) | |
|---|---|---|
| 2 | 1 | 4 |

Vorauflauf-Antidote-Test in Nährlösung

Es wird eine llewitt-Nährlösung hergestellt, die die nachstehend angegebene Menge Herbizid sowie 10 ppm des zu prüfenden Antidotes enthält.

Man verwendet Kultursamen, der in der angegebenen Prüfkonzentration von dem eingesetzten Herbizid erwartungsgemäss geschädigt werden sollte und sät ihn in gekörntes Zonolith (= expandiertes Vermikulit), das sich in einem an der Unterseite durchlöcherten Plastik-Blumentopf (oberer Ø 6 cm) befindet. Dieser wird in einen zweiten durchsichtigen Plastik-Blumentopf (oberer Ø 7 cm) gestellt, in dem sich ca. 50 ml der mit Herbizid und Antidote vorbereiteten Nährlösung befinden, die nunmehr kapillar im Füllmaterial des kleineren Topfes aufsteigt und Samen und keimende Pflanze benetzt. Täglich wird der Flüssigkeitsverlust mit reiner Hewitt-Nährlösung auf 50 ml ergänzt. 3 Wochen nach Testbeginn wird nach einer linearen Skala von 1 bis 9 ausgewertet, wobei 1 totale Pflanzenschädigung und 9 unbeeinträchtiger Gesundheitszustand bedeuten. Die parallel angewandte Kontroll-Lösung enthält keinen Antidote-Zusatz.

Man verwendet :

1) 4 ppm Prometryn = 2,4-bis(Isopropylamino)-6-methylthio-s-triazin in Sorghum-Hirse der Sorte « Funk G-522 ».

2) 4 ppm 4-Aethylamino-6-tert.butylamino-2-chlor-s-triazin in Weizen der Sorte « Farnese ».

3) 4 ppm α-[4-(p-Trifluormethylphenoxy)-phenoxy]-propionsäure-n-butoxyäthylester in Gerste der Sorte « Mazurka ».

4) 5 ppm Metolachlor = N-(1-Methyl-2-methoxy-äthyl)-N-chloracetyl-2-äthyl-6-methylanilin in Sorghum-Hirse der Sorte « Funk G-522 ».

Mit den Verbindungen der Formel I erhält man folgendes Resultat :

| Testvariante | Verb. Nr. | Note des Herbizideinflusses (ohne/mit Antidote) | |
|---|---|---|---|
| 4 | 13 | 4 | 6 |
| 4 | 14 | 4 | 7 |

Antidote Test Samenquellung (Seed Soaking)

Reissamen der Sorte IR 8 werden während 48 Stunden mit Lösungen der Testsubstanzen von 10 oder 100 ppm getränkt. Anschliessend werden die Samen etwa 2 Stunden trocknen gelassen, bis sie nicht mehr kleben. Rechteckige Plastiktöpfe (8 × 8 cm, 10 cm Höhe) werden bis 2 cm unter den Rand mit sandigem Lehm gefüllt. 4 g Samen werden pro Topf gesät und nur ganz schwach gedeckt (etwa Durchmesser des Samenkorns). Die Erde wird in einem feuchten (nicht sumpfigen) Zustand gehalten. Dann wird das Herbizid N-(1-Methyl-2-methoxyäthyl)-N-chloracetyl-2-äthyl-6-methylanilin in verdünnter Lösung und in einer Menge appliziert, die umgerechnet 1,5 kg AS/ha entspricht. 7 und 18 Tage nach dem Verpflanzen wird nach einer linearen Skala von 1 bis 9 ausgewertet, wobei 1 totale Pflanzenschädigung und 9 unbeeinträchtigter Gesundheitszustand bedeuten.

Wie aus der folgenden Tabelle ersichtlich, vermochten Verbindungen der Formel I in diesem Test die Reiskeimlinge vor der phytotoxischen Wirkung des verwendeten Herbizides zu schützen.

| Verb. Nr. | Konzentration | Note des Herbizideinflusses (ohne/mit Antidote) | |
|---|---|---|---|
| 4 | 100 ppm | 3 | 6 |
| | 10 ppm | 3 | 6 |
| 13 | 100 ppm | 3 | 6 |
| | 10 ppm | 3 | 7 |
| 15 | 10 ppm | 3 | 5 |
| 14 | 10 ppm | 3 | 6 |

Nachauflauf-Antidote-Test in Nährlösung

Allgemeine Methodik :

Man füllt kleine Plastik-Blumentöpfe (oberer Ø 6 cm), die an der Unterseite durchlöchert sind, mit gekörntem Zonolith und sät den Kultursamen ein. Dann wir der Topf in einen zweiten durchsichtigen Plastik-Blumentopf (oberer Ø 7 cm) gestellt, in dem sich 50 ml Wasser befinden, das kapillar aufsteigt und den Samen benetzt. Ab 5. Tag wird der laufende Wasserverlust mit Hewitt-Nährlösung ausgeglichen. Ab 15. Tag, wenn sich die Kulturpflanze im 11/2- bis 2-Blattstadium befindet, wird in die wieder auf 50 ml ergänzte Nährlösung

10 ppm des zu prüfenden Antidotes + die unten angegebene Menge Herbizid

zugegeben. Ab 16. Tag wird der Flüssigkeitsverlust wieder durch reine Hewitt-Nährlösung ausgeglichen. Während der gesamten Testdauer beträgt die Temperatur 20-23 °C bei einer relativen Luftfeuchtigkeit von 60-70 %.

3 Wochen nach Zugabe des Herbizids und des Antidotes erfolgt die Auswertung nach einer linearen Skala von 1 bis 9, wobei 1 totale Pflanzenschädigung und 9 unbeeinträchtigter Gesundheitszustand bedeuten.

Testvarianten :

1) 8 ppm α[4-(3,5-Dichlorpyridyl-2-oxy)-phenoxy]-propionsäure-propargylthioloester in Weizen der Sorte « Zenith ».

2) 4 ppm 4-Aethylamino-6-tert.butylamino-2-chlor-s-triazin in Weizen der Sorte « Zenith ».

3) 2 ppm α-[4-(p-Trifluormethylphenoxy)-phenoxy]-propionsäure-n-butoxyäthylester in Mais der Sorte « Orla ».

4) 8 ppm α-[4-(p-Trifluormethylphenoxy)-phenoxy]-propionsäure-n-butoxyäthylester in Sorghum-Hirse der Sorte « Funk G-522 ».

5) 4 ppm Prometryn = 2,4-bis(Isopropylamino)-6-methylthio-s-triazin in Sorghum-Hirse der Sorte « Funk G-522 ».

Verbindung Nr. 5 erzielt bei diesen Versuchen folgende Antidote-Wirkung.

| Testvariante | Verb. Nr. | Note des Herbizideinflusses (ohne/mit Antidote) | |
|---|---|---|---|
| 1 | 5 | 4 | 7 |

Antidote-Wirkung bei getrennter Applikation (Antidote-Vorauflauf. Herbizid-Nachauflauf)

Allgemeine Methodik :

Kleine Blumentöpfe (oberer Ø 6 cm) werden mit sandiger Lehmerde gefüllt, in die die Kulturpflanze eingesät wird. Nach dem Bedecken des Samens sprüht man die als Antidote zu prüfende Substanz in verdünnter Lösung und in einer Menge auf die Oberfläche, die umgerechnet 4 kg AS/ha beträgt. Man hält bei 20-23 °C und 60-70 % relativer Luftfeuchtigkeit. Wenn die Pflanzen nach 10 Tagen das 2- bis 3-Blattstadium erreicht haben, werden sie, wie nachfolgend angegeben, mit der entsprechenden Menge Herbizid behandelt. 14 Tage nach Herbizid-Applikation wird nach einer linearen Skala von 1 bis 9 ausgewertet, wobei 1 totale Pflanzenschädigung und 9 unbeeinträchtigter Gesundheitszustand bedeuten. Als Kontrolle dienen Pflanzen ohne Antidote-Schutz.

Man verwendet :

1) 4,0 kg AS/ha Ametryn = 2-Aethylamino-4-isopropylamino-6-methylthio-s-triazin in Mais der Sorte « Orla 264 ».

2) 1,0 kg AS/ha Prometryn = 2,4-bis(Isopropylamino)-6-methylthio-s-triazin in Sorghum-Hirse der Sorte « Funk G-522 ».

3) 0,25 kg AS/ha α-[4-(p-Trifluormethylphenoxy)-phenoxy]-propionsäure-n-butoxyäthylester in Gerste der Sorte « Mazurka ».

| Testvariante | Verb. Nr. | Note des Herbizideinflusses (ohne/mit Antidote) | |
|---|---|---|---|
| 2 | 11 | 3 | 5 |
| 2 | 8 | 3 | 5 |
| 2 | 14 | 3 | 7 |
| 3 | 15 | 4 | 6 |

Antidote-Wirkung an verpflanztem Reis bei getrennter Applikation
(Antidote-Vorauflauf. Herbizid-Nachauflauf)

Plastiktöpfe (8 × 8 cm, 10 cm Höhe) werden bis 2 cm unter den Rand mit Erde im sumpfig-nassen Zustand gefüllt. Die als Antidote zu prüfende Substanz wird in verdünnter Lösung und in einer Menge auf die Oberfläche gesprüht, die 4 kg AS/ha entspricht. Reispflanzen der Sorte « IR-8 » werden im 11/2- bis 2-Blattstadium in die so vorbereiteten Töpfe verpflanzt. Am nächsten Tag wird der Wasserstand auf ca. 1,5

cm erhöht. 4 Tage nach Verpflanzung werden umgerechnet 0,75 kg AS/ha von 2-Aethylamino-4-(1,2-dimethyl-n-propylamino)-6-methylthio-s-triazin in Granulatform ins Wasser gegeben. Die Temperatur beträgt während der Versuchsdauer 26-28 °C, die relative Luftfeuchtigkeit 60-80 %. 20 Tage nach Herbizidbehandlung wird nach einer linearen Skala von 1 bis 9 ausgewertet, wobei 1 totale Pflanzenschädigung und 9 unbeeinträchtigter Gesundheitszustand bedeuten. Als Kontrolle dienen Pflanzen ohne Antidote-Schutz.

Mit den Verbindungen der Formel I wurden folgende Resultate erzielt.

| Verb. Nr. | Note des Herbizideinflusses (ohne/mit Antidote) | |
|---|---|---|
| 11 | 3 | 9 |
| 8 | 3 | 7 |
| 3 | 3 | 6 |
| 12 | 3 | 6 |
| 13 | 3 | 6 |

Die Verbindungen der Formel I können für sich allein oder zusammen mit den zu antagonisierenden Herbiziden sowie zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen wie z. B. natürlichen oder regenerierten mineralischen Stoffen, Lösungs-, Dispergier-, Netz-, Haft-, Verdickungs-, Binde- oder Düngemitteln.

Der Gehalt an Wirkstoff in handelsfähigen Mitteln liegt zwischen 0,01 bis 90 %.

Zur Applikation können die Verbindungen der Formel I in den folgenden Aufarbeitungsformen vorliegen (wobei die Gewichts-Prozentangaben in Klammern vorteilhafte Mengen an Wirkstoff darstellen) :

Feste Aufarbeitungsformen : Stäubemittel und Streumittel (bis zu 10 %) Granulate, Umhüllungsgranulate, Imprägnierungsgranulate, Pellets (Körner) (1 bis 80 %) ;

Flüssige Aufarbeitungsformen :

a) in Wasser dispergierbare Wirkstoffkonzentrate : Spritzpulver (wettable powders) und Pasten (25-90 % in der Handelspackung, 0,01 bis 15 % in gebrauchsfertiger Lösung) Emulsions- und Lösungskonzentrate (10 bis 50 % ; 0,01 bis 15 % in gebrauchsfertiger Lösung) ;

b) Lösungen (0,1 bis 20 %), z. B. für Beize ; Aerosole.

Die Wirkstoffe der Formel I vorliegender Erfindung können beispielsweise wie folgt formuliert werden :

Stäubemittel : Zur Herstellung eines a) 5 %igen und b) 2 %igen Stäubemittels werden die folgenden Stoffe verwendet :

a) 5 Teile Wirkstoff
95 Teile Talkum ;
b) 2 Teile Wirkstoff
1 Teil hochdisperse Kieselsäure,
97 Teile Talkum ;

Die Wirkstoffe werden mit den Trägerstoffen vermischt und vermahlen und können in dieser Form zur Anwendung verstäubt werden.

Granulat : Zur Herstellung eines 5 %igen Granulates werden die folgenden Stoffe verwendet :

5 Teile Wirkstoff
0,25 Teile Epichlorhydrin,
0,25 Teile Cetylpolyglykoläther,
3,50 Teile Polyäthylenglykol
91 Teile Kaolin (Korngrösse 0,3-0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht, und anschliessend wird das Aceton im Vakuum verdampft. Ein derartiges Mikrogranulat lässt sich vorteilhaft in Saatfurchen einarbeiten.

Spritzpulver : Zur Herstellung eines 70 %igen Spritzpulvers werden folgende Bestandteile verwendet :

70 Teile Wirkstoff
5 Teile Natriumdibutylnaphtylsulfonat,
3 Teile Naphthalinsulfonsäuren-Phenolsulfonsäuren-Formaldehyd-Kondensat 3 : 2 : 1,
10 Teile Kaolin,
12 Teile Champagne-Kreide ;

Die Wirkstoffe werden in geeigneten Mischern mit den Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnen und insbesondere zur Saatbeizung und Tauchbehandlung von Stecklingen verwenden lassen.

Emulgierbare Konzentrate : Zur Herstellung eines 25 %igen emulgierbaren Konzentrates werden folgende Stoffe verwendet :

25 Teile Wirkstoff
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol.

Aus solchen Konzentraten können durch Verdünnen mit Wasser Emulsionen jeder gewünschten Konzentration hergestellt werden, die besonders zur Saatbeizung und Tauchbehandlung von Jungpflanzen geeignet sind.

**Ansprüche** (für die Vertragsstaaten : BE, CH, DE, FR, GB, IT, NL)

1. Verwendung von Verbindungen der Formel I

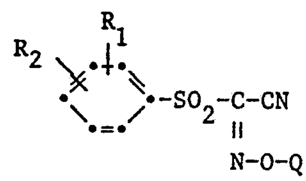

(I)

worin $R_1$ und $R_2$ unabhängig voneinander Wasserstoff, Halogen oder $C_1$-$C_4$-Alkyl bedeuten und Q die Acetamidgruppe, eine $C_1$-$C_4$-Alkoxycarbonylgruppe, eine $C_2$-$C_4$-Alkenylgruppe, eine N-($C_1$-$C_4$-Alkyl)-Carbamoylgruppe, eine Benzoylgruppe, die gegebenenfalls ein- bis dreifach durch gleiche oder verschiedene Substituenten aus der Gruppe Halogen, $C_1$-$C_4$-Alkyl und $C_1$-$C_4$-Alkoxy substituiert ist, oder eine 2-Furoylgruppe darstellt, zum Schützen von Kulturpflanzen vor der phytotoxischen Wirkung aggressiver Herbizide.

2. Verwendung einer Verbindung der Formel I gemäss Anspruch 1, in der $R_1$ Wasserstoff, Chlor, Methyl oder Aethyl und $R_2$ Wasserstoff bedeuten und Q $-CH_2CONH_2$, $-COOCH_3$, eine $C_2$-$C_4$-Alkenylgruppe, eine N-Methylcarbamoylgruppe, eine Benzoylgruppe, die durch zwei Chloratome und eine Methoxygruppe substituiert ist, oder eine 2-Furoylgruppe darstellt.

3. Verwendung gemäss Anspruch 1 der Verbindung der Formel

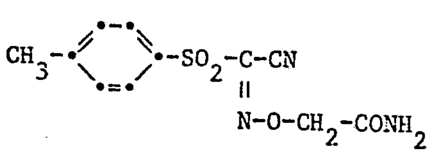

4. Verwendung gemäss Anspruch 1 der Verbindung der Formel

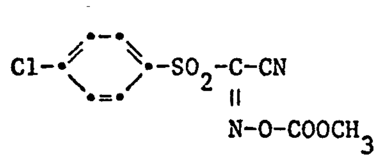

5. Verwendung gemäss Anspruch 1 der Verbindung der Formel

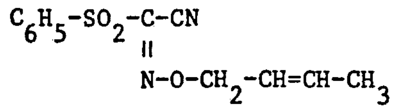

6. Verwendung gemäss Anspruch 1 der Verbindung der Formel

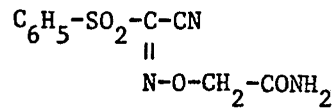

7. Verwendung gemäss Anspruch 1 der Verbindung der Formel

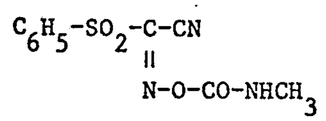

8. Verwendung gemäss Anspruch 1 der Verbindung der Formel

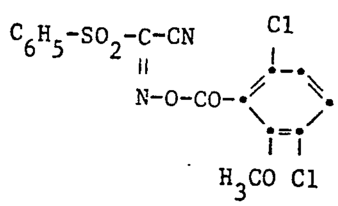

9. Verwendung gemäss Anspruch 1 der Verbindung der Formel

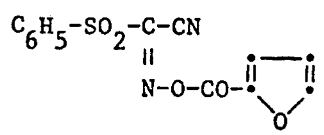

10. Mittel zum Schützen von Kulturpflanzen vor aggressiven Herbiziden enthaltend als Antidote ein Oximderivat der Formel I gemäss Anspruch 1, zusammen mit einem geeigneten Trägermaterial.

11. Mittel gemäss Anspruch 10 enthaltend ein Oximderivat der Formel I gemäss einem der Ansprüche 2 bis 9.

12. Antagonistisch wirkendes Mittel zum Schützen von Kulturpflanzen vor aggressiven Herbiziden enthaltend als Wirkstoffe a) das Herbizid sowie b) ein für Kulturpflanzen dazu als Antidote wirkendes Oximderivat der Formel I gemäss Anspruch 1 im Mischungsverhältnis 100 : 1 bis 1 : 5, vorzugsweise 20 : 1 bis 1 : 1.

13. Vermehrungsgut von Kulturpflanzen, behandelt mit einem Oximderivat der Formel I des Anspruchs 1.

14. Vermehrungsgut gemäss Anspruch 13, dadurch gekennzeichnet, dass es sich um Saatgut handelt.

15. Verbindung der Formel I

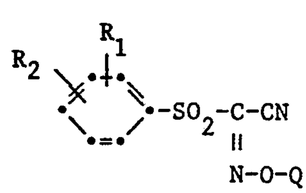

(I)

gemäss Anspruch 1, worin $R_1$ und $R_2$ unabhängig voneinander Wasserstoff, Halogen oder $C_1$-$C_4$-Alkyl bedeuten und Q die Acetamid-Gruppe, eine $C_1$-$C_4$-Alkoxycarbonylgruppe, eine $C_2$-$C_4$-Alkenylgruppe, eine N-($C_1$-$C_4$-Alkyl)-Carbamoylgruppe, eine Benzoylgruppe, die gegebenenfalls ein- bis dreifach durch gleiche oder verschiedene Substituenten aus der Gruppe Halogen, $C_1$-$C_4$-Alkyl und $C_1$-$C_4$-Alkoxy substituiert ist, oder eine 2-Furoylgruppe darstellt.

16. Verbindungen der Formel

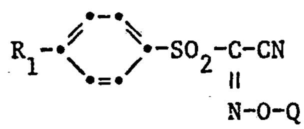

(I)

gemäss Anspruch 15, worin $R_1$ Wasserstoff, Chlor, Methyl oder Aethyl bedeutet und Q $-CH_2CONH_2$, $-COOCH_3$, eine $C_2$-$C_4$- Alkenylgruppen, eine N-Methylcarbamoylgruppe, eine Benzoylgruppe, die durch zwei Chloratome und eine Methoxygruppe substituiert ist, oder eine 2-Furoylgruppe darstellt.

17. Die Verbindung der Formel

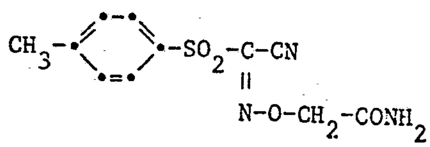

gemäss Anspruch 16.

18. Die Verbindung der Formel

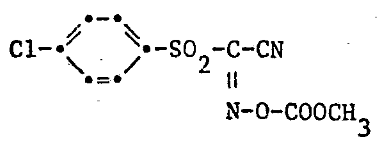

gemäss Anspruch 16.

19. Die Verbindung der Formel

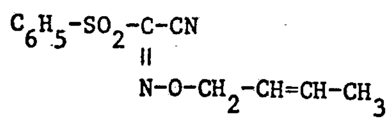

gemäss Anspruch 16.

20. Die Verbindung der Formel

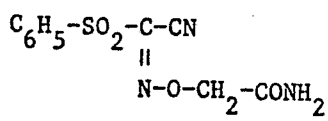

gemäss Anspruch 16.

21. Die Verbindung der Formel

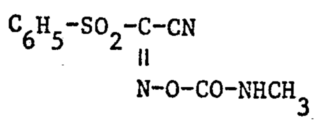

gemäss Anspruch 16.

22. Die Verbindung der Formel

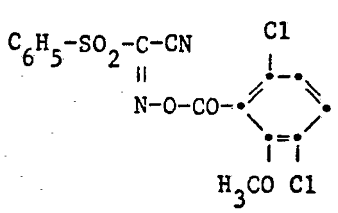

gemäss Anspruch 16.

23. Die Verbindung der Formel

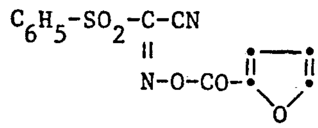

gemäss Anspruch 16.

**Ansprüche** (für den Vertragsstaat AT)

1. Verwendung von Verbindungen der Formel I

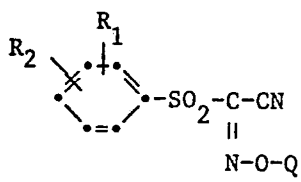

(I)

worin $R_1$ und $R_2$ unabhängig voneinander Wasserstoff, Halogen oder $C_1$-$C_4$-Alkyl bedeuten und Q die Acetamidgruppe, eine $C_1$-$C_4$-Alkoxycarbonylgruppe, eine $C_2$-$C_4$-Alkenylgruppe, eine N-($C_1$-$C_4$-Alkyl)-Carbamoylgruppe, eine Benzoylgruppe, die gegebenenfalls ein- bis dreifach durch gleiche oder verschiedene Substituenten aus der Gruppe Halogen, $C_1$-$C_4$-Alkyl und $C_1$-$C_4$-Alkoxy substituiert ist, oder eine 2-Furoylgruppe darstellt, zum Schützen von Kulturpflanzen vor der phytotoxischen Wirkung aggressiver Herbizide.

2. Verwendung einer Verbindung der Formel I gemäss Anspruch 1, in der $R_1$ Wasserstoff, Chlor, Methyl oder Aethyl und $R_2$ Wasserstoff bedeuten und Q —$CH_2CONH_2$, —$COOCH_3$, eine $C_2$-$C_4$-Alkenylgruppe, eine N-Methylcarbamoylgruppe, eine Benzoylgruppe, die durch zwei Chloratome und eine Methoxygruppe substituiert ist, oder eine 2-Furoylgruppe darstellt.

3. Verwendung gemäss Anspruch 1 der Verbindung der Formel

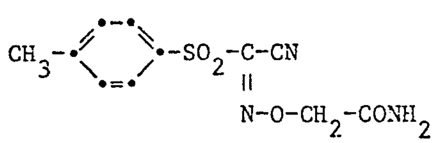

4. Verwendung gemäss Anspruch 1 der Verbindung der Formel

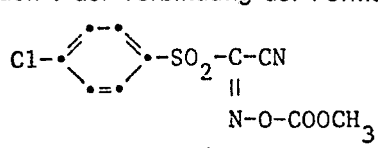

5. Verwendung gemäss Anspruch 1 der Verbindung der Formel

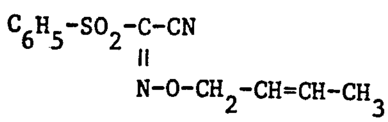

6. Verwendung gemäss Anspruch 1 der Verbindung der Formel

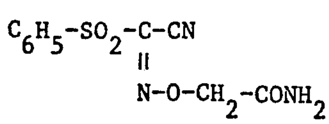

7. Verwendung gemäss Anspruch 1 der Verbindung der Formel

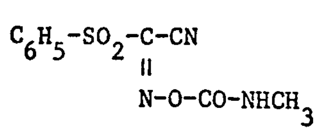

8. Verwendung gemäss Anspruch 1 der Verbindung der Formel

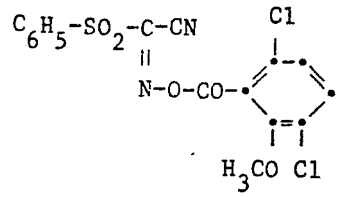

9. Verwendung gemäss Anspruch 1 der Verbindung der Formel

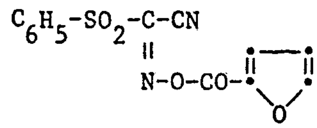

10. Mittel zum Schützen von Kulturpflanzen vor aggressiven Herbiziden enthaltend als Antidote ein Oximderivat der Formel I gemäss Anspruch 1, zusammen mit einem geeigneten Trägermaterial.

11. Mittel nach Anspruch 10, welches als Antidot eine Verbindung der Formel I

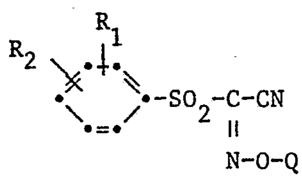

(I)

worin $R_1$ und $R_2$ unabhängig voneinander Wasserstoff, Halogen oder $C_1$-$C_4$-Alkyl bedeuten und Q die Acetamid-Gruppe, eine $C_1$-$C_4$-Alkoxycarbonylgruppe, eine $C_2$-$C_4$-Alkenylgruppe, eine N-($C_1$-$C_4$-Alkyl)-Carbamoylgruppe, eine Benzoylgruppe, die gegebenenfalls ein- bis dreifach durch gleiche oder verschiedene Substituenten aus der Gruppe Halogen, $C_1$-$C_4$-Alkyl und $C_1$-$C_4$-Alkoxy substituiert ist, oder eine 2-Furoylgruppe darstellt, enthält.

12. Mittel nach Anspruch 10, welches als Antidot eine Verbindung der Formel

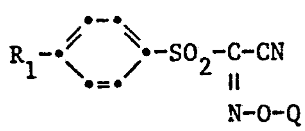

worin $R_1$ Wasserstoff, Chlor, Methyl oder Aethyl bedeutet und Q $-CH_2CONH_2$, $-COOCH_3$, eine $C_2$-$C_4$-Alkenylgruppe, eine N-Methylcarbamoylgruppe, eine Benzoylgruppe, die durch zwei Chloratome und eine Methoxygruppe substituiert ist, oder eine 2-Furoylgruppe darstellt, enthält.

13. Mittel nach Anspruch 10, welches als Antidot die Verbindung der Formel

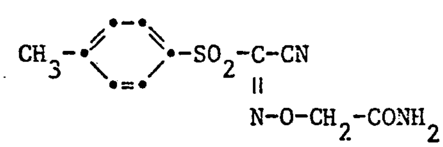

enthält.

14. Mittel nach Anspruch 10, welches als Antidot die Verbindung der Formel

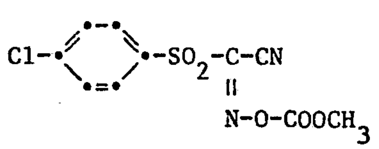

enthält.

15. Mittel nach Ansrpuch 10, welches als Antidot die Verbindung der Formel

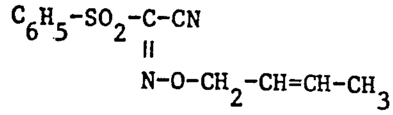

enthält.

16. Mittel nach Anspruch 10, welches als Antidot die Verbindung der Formel

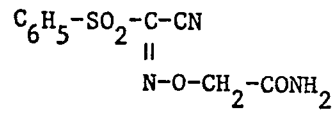

enthält.

17. Mittel nach Anspruch 10, welches als Antidot die Verbindung der Formel

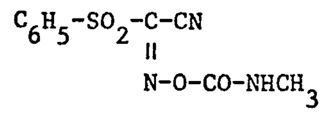

enthält.

18. Mittel nach Anspruch 10, welches als Antidot die Verbindung der Formel

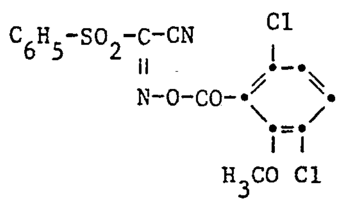

enthält.

19. Mittel nach Anspruch 10, welches als Antidot die Verbindung der Formel

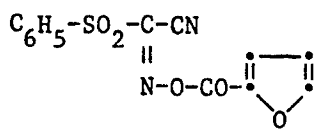

enthält.

20. Antagonistisch wirkendes Mittel zum Schützen von Kulturpflanzen vor aggressiven Herbiziden enthaltend als Wirkstoffe a) Herbizid sowie b) ein für Kulturpflanzen dazu als Antidote wirkendes Oximderivat der Formel I gemäss Anspruch 1 im Mischungsverhältnis 100 : 1 bis 1 : 5, vorzugsweise 20 : 1 bis 1 : 1.

21. Vermehrungsgut von Kulturpflanzen, behandelt mit einem Oximderivat der Formel I des Anspruchs 1.

22. Vermehrungsgut gemäss Anspruch 21, dadurch gekennzeichnet, dass es sich um Saatgut handelt.

**Claims** (for the Contracting States : BE, CH, DE, FR, GB, IT, NL)

1. Use of a compound of formula I

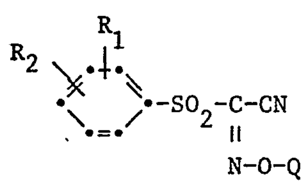 (I)

wherein each of $R_1$ and $R_2$ independently of the other is hydrogen, halogen or $C_1$-$C_4$alkyl and Q is the acetamide group, a $C_1$-$C_4$alkoxycarbonyl group, a $C_2$-$C_4$alkenyl group, an N-($C_1$-$C_4$alkyl)carbamoyl

group, a benzoyl group which is unsubstituted or substituted by one to three identical or different substituents selected from the group consisting of halogen, $C_1$-$C_4$alkyl and $C_1$-$C_4$alkoxy, or is a 2-furoyl group, for protecting cultivated plants from the phytotoxic action of aggressive herbicides.

2. Use of a compound of formula I according to claim 1, wherein $R_1$ is hydrogen, chlorine, methyl or ethyl and $R_2$ is hydrogen and Q is $-CH_2CONH_2$, $COOCH_3$, a $C_2$-$C_4$alkenyl group, an N-methylcarbamoyl group, a benzoyl group which is substituted by two chlorine atoms and a methoxy group, or is a 2-furoyl group.

3. Use according to claim 1 of a compound of the formula

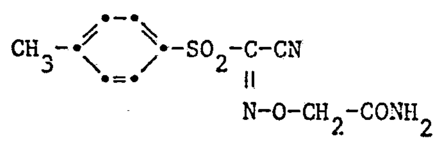

4. Use according to claim 1 of a compound of the formula

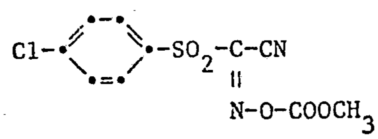

5. Use according to claim 1 of a compound of the formula

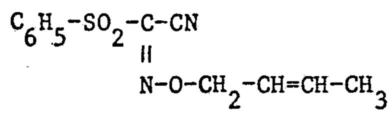

6. Use according to claim 1 of a compound of the formula

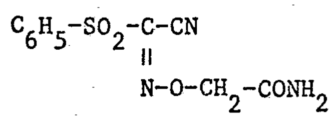

7. Use according to claim 1 of a compound of the formula

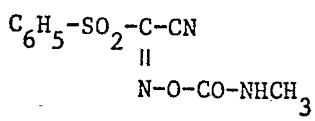

8. Use according to claim 1 of a compound of the formula

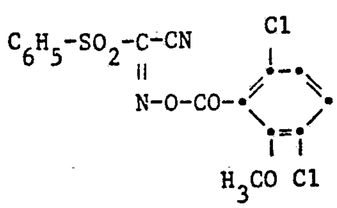

9. Use according to claim 1 of a compound of the formula

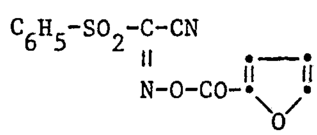

10. A composition for protecting cultivated plants from aggressive herbicides, which composition contains as antidote an oxime derivative of formula I according to claim 1, together with a suitable carrier.

11. A composition according to claim 10, which contains an oxime derivative of formula I according to any one of claims 2 to 9.

12. An antagonistic composition for protecting cultivated plants from the effects of aggressive herbicides, which composition contains a) the herbicide b) an oxime derivative of formula I according to claim 1, which acts as an antidote for the cultivated plants, in the ratio of 100 : 1 to 1 : 5, preferably 20 : 1 to 1 : 1.

13. Propagation products of cultivated plants treated with an oxime derivate of formula I according to claim 1.

14. Propagation products according to claim 13, which are seeds.

15. A compound of formula I

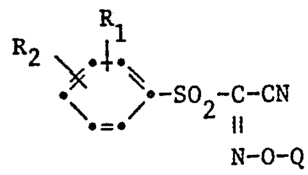

(I)

according to claim 1, wherein each of $R_1$ and $R_2$ independently of the other is hydrogen, halogen or $C_1$-$C_4$alkyl and Q is the acetamide group, a $C_1$-$C_4$alkoxycarbonyl group, a $C_2$-$C_4$alkenyl group, an N-($C_1$-$C_4$alkyl)carbamoyl group, a benzoyl group which is unsubstituted or substituted by one to three identical or different substituents selected from the group consisting of halogen, $C_1$-$C_4$alkyl and $C_1$-$C_4$alkoxy, or is a 2-furoyl group.

16. A compound of the formula

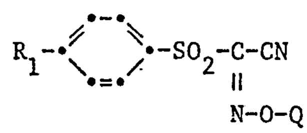

according to claim 15, wherein $R_1$ is hydrogen, chlorine, methyl or ethyl and Q is $-CH_2CONH_2$, $COOCH_3$, a $C_2$-$C_4$alkenyl group, an N-methylcarbamoyl group, a benzoyl group which is substituted by two chlorine atoms and a methoxy group, or is a 2-furoyl group.

17. The compound of the formula

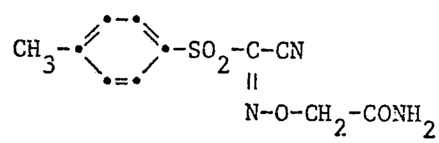

according to claim 16.

18. The compound of the formula

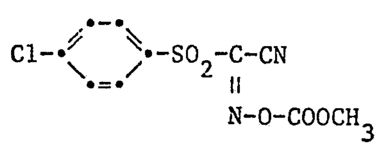

according to claim 16.

19. The compound of the formula

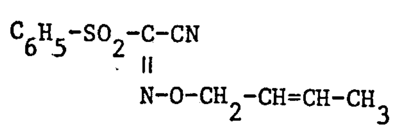

according to claim 16.

20. The compound of the formula

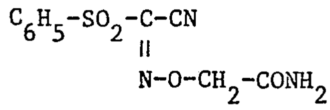

according to claim 16.

21. The compound of the formula

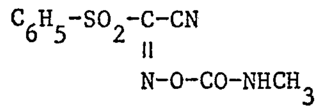

according to claim 16.

22. The compound of the formula

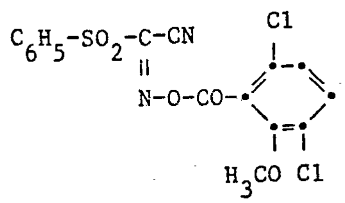

according to claim 16.

23. The compound of the formula

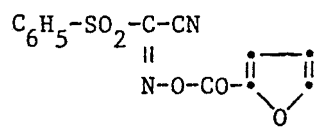

according to claim 16.

**Claims** (for the Contracting State AT)

1. Use of a compound of formula I

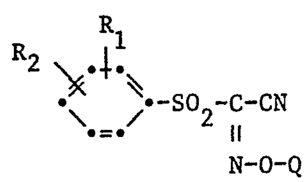

(I)

wherein each of $R_1$ and $R_2$ independently of the other is hydrogen, halogen or $C_1$-$C_4$alkyl and Q is the acetamide group, a $C_1$-$C_4$alkoxycarbonyl group, a $C_2$-$C_4$alkenyl group, an N-($C_1$-$C_4$alkyl)carbamoyl group, a benzoyl group which is unsubstituted or substituted by one to three identical or different substituents selected from the group consisting of halogen, $C_1$-$C_4$alkyl and $C_1$-$C_4$alkoxy, or is a 2-furoyl group, for protecting cultivated plants from the phytotoxic action of aggressive herbicides.

2. Use of a compound of formula I according to claim 1, wherein $R_1$ is hydrogen, chlorine, methyl or ethyl and $R_2$ is hydrogen and Q is —$CH_2CONH_2$, $COOCH_3$, a $C_2$-$C_4$alkenyl group, an N-methylcarbamoyl group, a benzoyl group which is substituted by two chlorine atoms and a methoxy group, or is a 2-furoyl group.

3. Use according to claim 1 of a compound of the formula

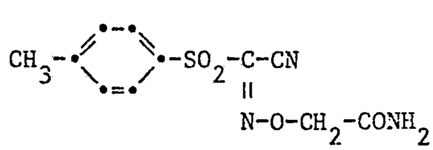

4. Use according to claim 1 of a compound of the formula

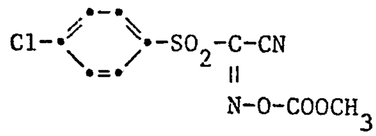

5. Use according to claim 1 of a compound of the formula

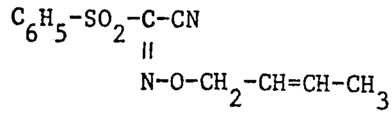

6. Use according to claim 1 of a compound of the formula

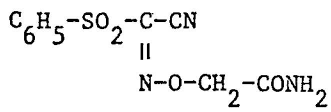

7. Use according to claim 1 of a compound of the formula

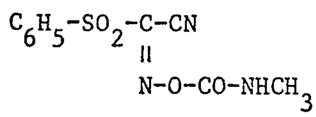

8. Use according to claim 1 of a compound of the formula

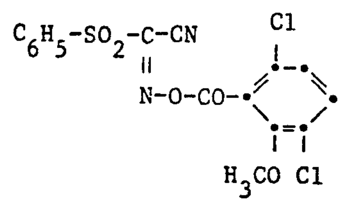

9. Use according to claim 1 of a compound of the formula

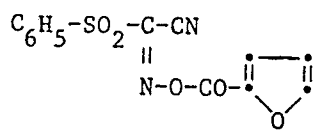

10. A composition for protecting cultivated plants from aggressive herbicides, which composition contains as antidote an oxime derivative of formula I according to claim 1, together with a suitable carrier.

11. A composition according to claim 10, which contains as antidote a compound of the formula I

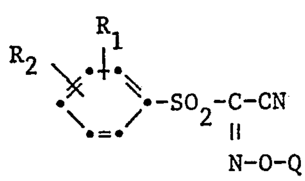

(I)

wherein each of $R_1$ and $R_2$ independently of the other is hydrogen, halogen or $C_1$-$C_4$alkyl and Q is the acetamide group, a $C_1$-$C_4$alkoxycarbonyl group, a $C_2$-$C_4$alkenyl group, an N-($C_1$-$C_4$alkyl)carbamoyl group, a benzoyl group which is usubstituted or substituted by one to three identical or different substituents selected from the group consisting of halogen, $C_1$-$C_4$alkyl and $C_1$-$C_4$alkoxy, or is a 2-furoyl group.

12. A composition according to claim 10, which contains as antidote a compound of the formula

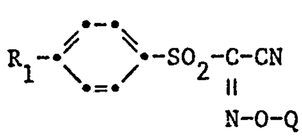

wherein $R_1$ is hydrogen, chlorine, methyl or ethyl and Q is $-CH_2CONH_2$, $COOCH_3$, a $C_2$-$C_4$alkenyl group, an N-Methylcarbamoyl group, a benzoyl group which is substituted by two chlorine atoms and a methoxy group, or is a 2-furoyl group.

13. A composition according to claim 10, which contains as antidote the compound of the formula

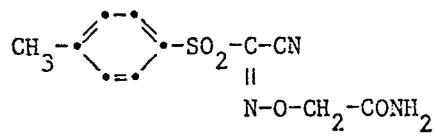

14. A composition according to claim 10, which contains as antidote the compound of the formula

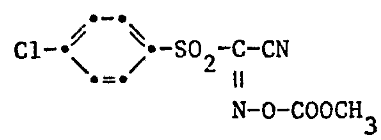

15. A composition according to claim 10, which contains as antidote the compound of the formula

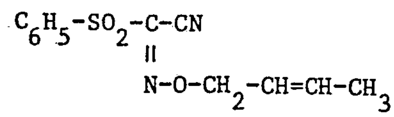

16. A composition according to claim 10, which contains as antidote the compound of the formula

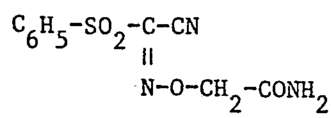

17. A composition according to claim 10, which contains as antidote the compound of the formula

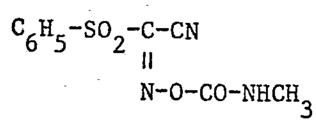

18. A composition according to claim 10, which contains as antidote the compound of the formula

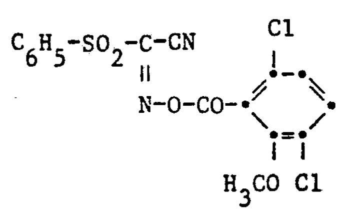

19. A composition according to claim 10, which contains as antidote the compound of the formula

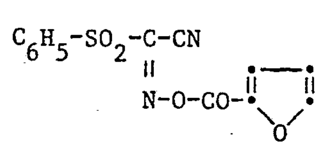

20. An antagonistic composition for protecting cultivated plants from the effects of aggressive herbicides, which composition contains a) the herbicide and b) an oxime derivative of formula I according to claim 1, which acts as an antidote for the cultivated plants, in the ratio of 100 : 1 to 1 : 5, preferably 20 : 1 to 1 : 1.

21. Propagation products of cultivated plants treated with an oxime derivative of formula I according to claim 1.

22. Propagation products according to claim 21, which are seeds.

**Revendications** (pour les Etats contractants : BE, CH, DE, FR, GB, IT, NL)

1. Utilisation des composés de formule I

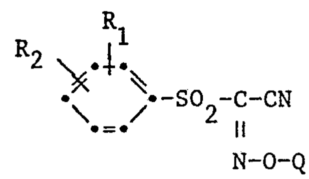

(I)

dans laquelle $R_1$ et $R_2$ représentent, indépendamment l'un de l'autre, l'hydrogène, un halogène ou un groupe alkyle en $C_1$-$C_4$ et Q représente le groupe acétamido, un groupe (alcoxy en $C_1$-$C_4$)-carbonyle, un groupe alcényle en $C_2$-$C_4$, un groupe N-(alkyle en $C_1$-$C_4$)-carbamoyle, un groupe benzoyle, qui porte le cas échéant 1 à 3 substituants identiques ou différents pris dans le groupe formé par les halogènes, les groupes alkyle en $C_1$-$C_4$ et alcoxy en $C_1$-$C_4$, ou un groupe 2-furoyle, pour la protection des végétaux cultivés contre l'activité phytotoxique des herbicides agressifs.

2. Utilisation d'un composé de formule I selon la revendication 1, dans laquelle $R_1$ représente l'hydrogène, le chlore, un groupe méthyle ou éthyle et $R_2$ représente l'hydrogène, et Q représente un groupe $—CH_2CONH_2$, $—COOCH_3$, un groupe alcényle en $C_2$-$C_4$, un groupe N-méthylcarbamoyle, un groupe benzoyle, qui est substitué par deux atomes de chlore et un groupe métoxy, ou un groupe furoyle.

3. Utilisation selon la revendication 1 du composé de formule

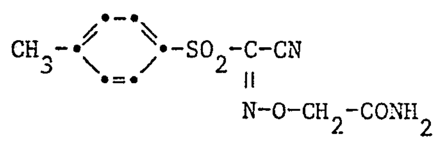

4. Utilisation selon la revendication 1 du composé de formule

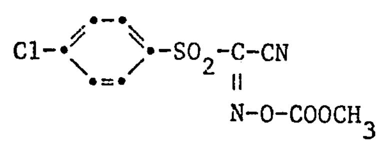

5. Utilisation selon la revendication 1 du composé de formule

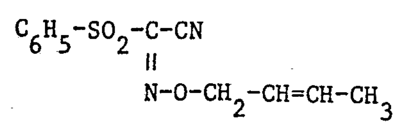

6. Utilisation selon la revendication 1 du composé de formule

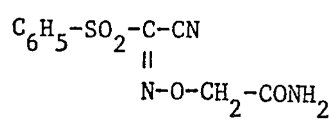

7. Utilisation selon la revendication 1 du composé de formule

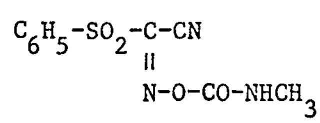

8. Utilisation selon la revendication 1 du composé de formule

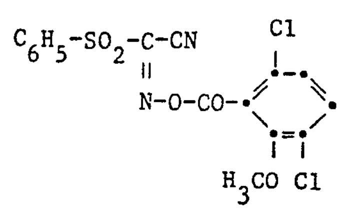

9. Utilisation selon la revendication 1 du composé de formule

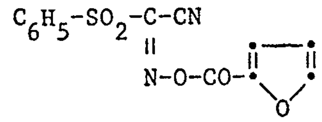

10. Produit pour la protection des végétaux cultivés contre les herbicides agressifs, contenant en tant qu'antidote un dérivé d'oxime de formule I selon la revendication 1 avec un véhicule approprié.

11. Produit selon la revendication 10, contenant un dérivé d'oxime de formule I selon l'une des revendications 2 à 9.

12. Produit à activité antagoniste pour la protection des végétaux cultivés contre les herbicides agressifs, contenant, en tant que substances actives a) l'herbicide, et b) un dérivé d'oxime de formule I selon la revendication 1 agissant comme antidote à l'égard de cet herbicide pour les végétaux cultivés, dans des proportions relatives de mélange de 100 : 1 à 1 : 5, de préférence de 20 : 1 à 1 : 1.

13. Produit de multiplication de végétaux cultivés, traité par un dérivé d'oxime de formule I de la revendication 1.

14. Produit de multiplication selon la revendication 13, caractérisé en ce qu'il consiste en semences.

15. Composé de formule I

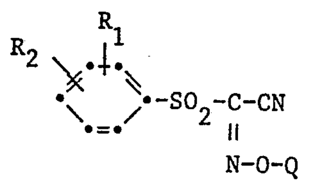

(I)

selon la revendication 1, dans laquelle $R_1$ et $R_2$ représentent, indépendamment l'un de l'autre, l'hydrogène, un halogène ou un groupe alkyle en $C_1$-$C_4$ et Q représente le groupe acétamido, un groupe (alcoxy en $C_1$-$C_4$)-carbonyle, un groupe alcényle en $C_2$-$C_4$, un groupe N-(alkyle en $C_1$-$C_4$)-carbamoyle, un groupe benzoyle, portant éventuellement 1 à 3 substituants identiques ou différents du groupe des halogènes, des groupes alkyle en $C_1$-$C_4$ et alcoxy en $C_1$-$C_4$, ou un groupe 2-furoyle.

16. Composés de formule

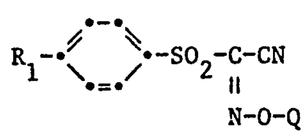

selon la revendication 15, dans laquelle $R_1$ représente l'hydrogène, le chlore, un groupe méthyle ou éthyle et Q représente un groupe $—CH_2CONH_2$, $—COOCH_3$, un groupe alcényle en $C_2$-$C_4$, un groupe N-méthylcarbamoyle, un groupe benzoyle, substitué par deux atomes de chlore et un groupe méthoxy, ou un groupe 2-furoyle.

17. Le composé de formule

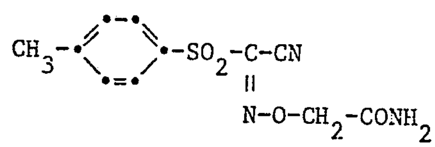

selon la revendication 16.

18. Le composé de formule

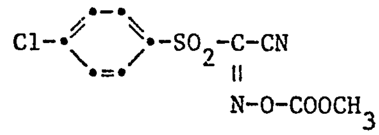

selon la revendication 16.

19. Le composé de formule

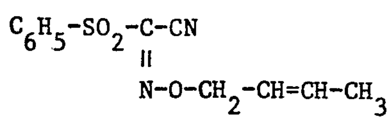

selon la revendication 16.

20. Le composé de formule

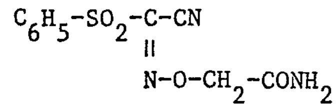

selon la revendication 16.

21. Le composé de formule

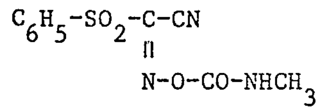

selon la revendication 16.

22. Le composé de formule

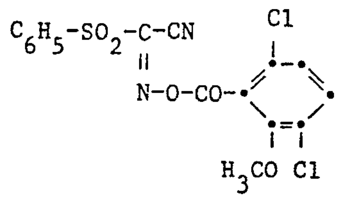

selon la revendication 16.

23. Le composé de formule

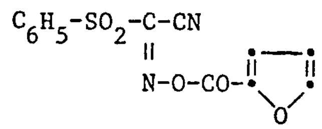

selon la revendication 16.

**Revendications** (pour l'Etat contractant AT)

1. Utilisation des composés de formule I

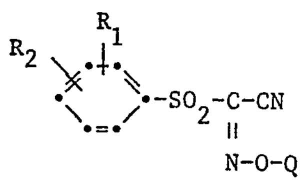 (I)

dans laquelle $R_1$ et $R_2$ représentent, indépendamment l'un de l'autre, l'hydrogène, un halogène ou un groupe alkyle en $C_1$-$C_4$ et Q représente le groupe acétamido, un groupe (alcoxy en $C_1$-$C_4$)-carbonyle, un groupe alcényle en $C_2$-$C_4$, un groupe N-(alkyle en $C_1$-$C_4$)-carbamoyle, un groupe benzoyle, qui porte le cas échéant 1 à 3 substituants identiques ou différents pris dans le groupe formé par les halogènes, les groupes alkyle en $C_1$-$C_4$ et alkoxy en $C_1$-$C_4$, ou un groupe 2-furoyle, pour la protection des végétaux cultivés contre l'activité phytotoxique des herbicides agressifs.

2. Utilisation d'un composé de formule I selon la revendication 1, dans laquelle $R_1$ représente l'hydrogène, le chlore, un groupe méthyle ou éthyle et $R_2$ représente l'hydrogène, et Q représente un groupe $—CH_2CONH_2$, $—COOCH_3$, un groupe alcényle en $C_2$-$C_4$, un groupe N-méthylcarbamoyle, un groupe benzoyle, qui est substitué par deux atomes de chlore et un groupe méthoxy, ou un groupe furoyle.

3. Utilisation selon la revendication 1 du composé de formule

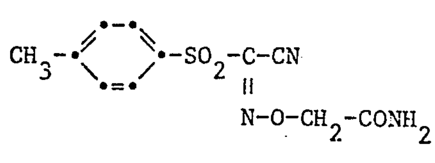

4. Utilisation selon la revendication 1 du composé de formule

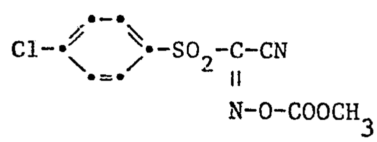

5. Utilisation selon la revendication 1 du composé de formule

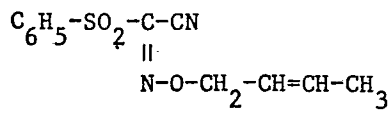

6. Utilisation selon la revendication 1 du composé de formule

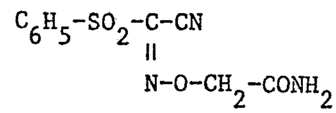

7. Utilisation selon la revendication 1 du composé de formule

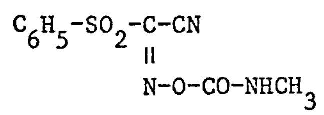

8. Utilisation selon la revendication 1 du composé de formule

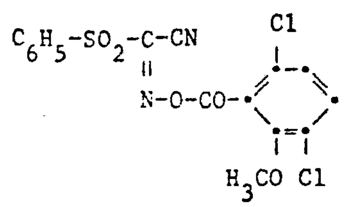

9. Utilisation selon la revendication 1 du composé de formule

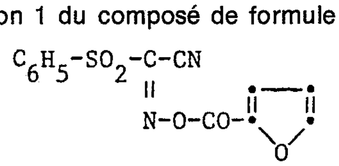

10. Produit pour la protection des végétaux cultivés contre les herbicides agressifs, contenant en tant qu'antidote un dérivé d'oxime de formule I selon la revendication 1 avec un véhicule approprié.

11. Produit selon la revendication 10, contenant en tant qu'antidote un composé de formule I

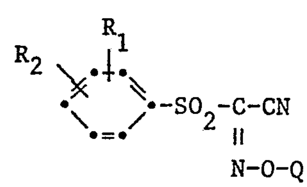 (I)

dans laquelle $R_1$ et $R_2$ représentent, indépendamment l'un de l'autre, l'hydrogène, un halogène ou un groupe alkyle en $C_1$-$C_4$, Q représente le groupe acétamido, un groupe (alcoxy en $C_1$-$C_4$)-carbonyle, un groupe alcényle en $C_2$-$C_4$, un groupe N-(alkyle en $C_1$-$C_4$)-carbamoyle, un groupe benzoyle, portant le cas échéant 1 à 3 substituants identiques ou différents du groupe des halogènes, des groupes alkyle en $C_1$-$C_4$ et alcoxy en $C_1$-$C_4$ ou un groupe 2-furoyle.

12. Produit selon la revendication 10, contenant en tant qu'antidote un composé de formule

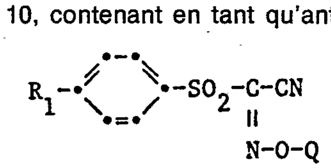

dans laquelle $R_1$ représente l'hydrogène, le chlore, un groupe méthyle ou éthyle et Q représente un groupe $-CH_2CONH_2$, $-COOCH_3$, un groupe alcényle en $C_2$-$C_4$, un groupe N-méthylcarbamoyle, un groupe benzoyle substitué par 2 atomes de chlore et un groupe méthoxy, ou un groupe 2-furoyle.

13. Produit selon la revendication 10, contenant en tant qu'antidote le composé de formule

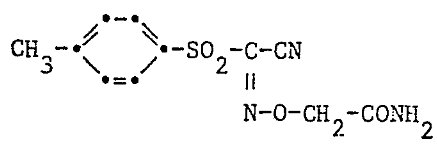

14. Produit selon la revendication 10, contenant en tant qu'antidote le composé de formule

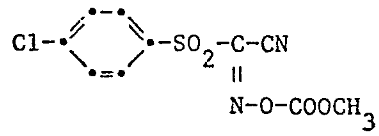

15. Produit selon la revendication 10, contenant en tant qu'antidote le composé de formule

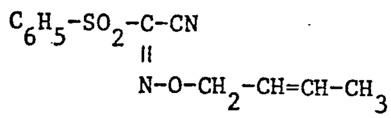

16. Produit selon la revendication 10, contenant en tant qu'antidote le composé de formule

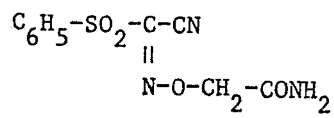

17. Produit selon la revendication 10, contenant en tant qu'antidote le composé de formule

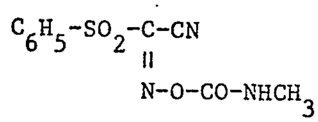

18. Produit selon la revendication 10, contenant en tant qu'antidote le composé de formule

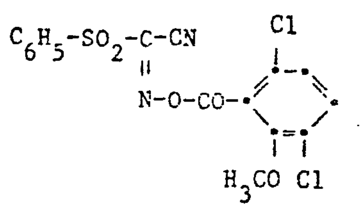

19. Produit selon la revendication 10, contenant en tant qu'antidote le composé de formule

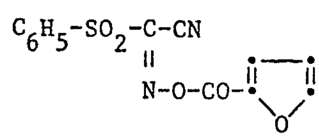

20. Produit à activité antagoniste pour la protection des végétaux cultivés contre les herbicides agressifs, contenant en tant que substances actives a) l'herbicide et b) un dérivé d'oxime de formule I selon la revendication 1 agissant comme antidote à l'égard de cet herbicide pour les végétaux cultivés, dans les proportions relatives de mélange de 100 : 1 à 1 : 5, de préférence de 20 : 1 à 1 : 1.

21. Produit de multiplication de végétaux cultivés, traité par un dérivé d'oxime de formule I de la revendication 1.

22. Produit de multiplication selon la revendication 21, caractérisé en ce qu'il consiste en semences.